# EUROPEAN PATENT APPLICATION

(11) **EP 0 948 968 A1**
(43) Date of publication of application: **13.10.1999**
(21) Application number: 98201154.6
(22) Date of filing: 09.04.1998
(51) Int. Cl.: A61K 31/715

(54) **Therapeutic agents interfering in neutrophil migration**

(71) Applicant: ACADEMISCH ZIEKENHUIS UTRECHT, 3584 CX Utrecht (NL)
(72) Inventor: The designation of the inventor has not yet been filed
(74) Representative: Smulders, Theodorus A.H.J., Ir.

(57) **Abstract**

Glucuronoxylomannan or a functional equivalent thereof for use as a pharmaceutical.

## Description

The present invention relates to the field of diseases involving the immune sytem, in particular diseases involving neutrophil migration. A number of serious illnesses involve neutrophil transmigration at one stage or the other. Such diseases include acute traumas (especially trauma to the brain), infections and other inflammatory diseases (such as autoimmune diseases). A very important disease for which no successful drug has been developed sofar is bacterial meningitis.

Bacterial meningitis is a serious illness, affecting annually in the United States about 3 individuals per 100,000. The case fatality is estimated at 25 %, and a substantial percentage of the survivors will suffer permanent neurological impairment. Typically, a patient presents with serious clinical symptoms and a high leukocyte count in their cerebrospinal fluid (CSF). Extensive literature evidence suggests that these leukocytes, which are mainly neutrophil leukocytes, play an important adverse role in the pathogenesis of neurological damage.

It has been demonstrated that a high influx of neutrophil leukocytes into the central nervous system (CNS) correlates with an unfavorable prognosis for neurological recovery. Therefore, several attempts have been made to reduce the inflammatory response. So far, the only studies published with adjuvant anti-inflammatory treatment in human bacterial meningitis, are clinical trials with corticosteroids added to the antibiotic regimen. At least one of the proposed effects of corticosteroids involves the interference of neutrophil migration, although other mechanisms may play a role as well. Results of these clinical trials have been promising though controversial, and only in pediatric cases the routine use of corticosteroid therapy is currently recommended. In adult bacterial meningitis, more evidence of a positive effect of adjuvant corticosteroid treatment is needed.

As stated before bacterial meningitis is not the only clinical situation where the influx of neutrophils has been associated with tissue damage. In serious brain injury (brain neurotrauma) and in ischemic events such as cerebrovascular ischemia, myocardial infarction, intestinal ischemia, pulmonary ischemia, and skeletal muscular ischemia, tissue reperfusion accompanied by neutrophil influx in tissue has repeatedly been correlated to tissue damage. Investigations in the role of neutrophils in non-infectious CNS damage have currently been intensified, since other therapeutical options in this field are very limited.
Experimental animal models have been established to study various anti-inflammatory agents, such as superoxide dismutase, transforming growth factor β, antibodies against adhesion molecules such as CD18 (IB4) and ICAM, polysaccharides and cyclooxygenase inhibitors. Many of these strategies aim either directly at the reduction of the neutrophil influx or at (the release of) toxic neutrophil products into the CNS. Even though the results look promising in animals, the effectiveness of these agents to reduce the migration of neutrophils into the CNS in the human situation remains to be established, and there is a considerable concern about potential side-effects such as an adverse reaction to animal proteins (antibodies) and an increased susceptibility to infection through substantial interference with neutrophil function. Therefore, to our knowledge, the agents mentioned have not yet resulted in sucessful therapeutic tools that can be applied in the human situation. Thus, there is still an urgent need for new therapeutic means especially in CNS disease.

The present invention provides a novel means for suppressing neutrophil migration and thus a novel means for preventing damage resulting from neutrophil migration. The invention provides Glucuronoxylomannan or a functional equivalent thereof for use as a pharmaceutical.

In particular the invention provides the use of glucuronoxylmannan or a functional equivalent thereof in the preparation of a medicament for the treatment of a pathological condition or a disease involving neutrofil migration.

In inflammation, soluble factors called chemokines have been identified, which attract various subtypes of leukocytes. The chemokine IL-8 is a potent chemoattractant for neutrophils {25}. As expected, patients with bacterial meningitis have high levels of IL-8 as well as an increased number of neutrophils in their cerebrospinal fluid (CSF). We recently described that patients who suffer from a fungal meningitis caused by *Cryptococcus neoformans*, whose CSF typically contains very little leukocytes, had high levels of IL-8 in their CSF {26}. Therefore, in cryptococcal meningitis, an agent must be present that interferes with neutrophil migration towards IL-8. Patients with cryptococcal infection have high titers of the fungal capsular polysaccharide glucuronoxylomannan (GXM) in both serum and CSF during infection {27,28}. Cryptococcal polysaccharides have been shown to interfere with neutrophil movement {29, 30, 31, 32}. Moreover, we demonstrated *in vitro* that GXM also interferes with neutrophil migration (chemotaxis) towards IL-8, if GXM is added directly to the leukocytes {26}. We also demonstrated that a low CSF leukocyte cell count in patients with cryptococcal meningitis is significantly correlated to a high GXM titer in serum (relative to the GXM titer in CSF) (Lipovsky, in preparation).

The exact mechanism how GXM interferes with neutrophil migration in cryptococcosis is not known. GXM consists of an α-1,3-linked polymannose backbone with O-acetyl substituents and β-linked monomeric branches of xylose and glucuronic acid {72}. GXM has been shown to attract neutrophils itself {30,26}. Moreover, GXM has been shown to interfere with molecules that are essential in neutrophil-endothelial interaction, for instance: it can bind to CD18 (which is the β subunit of leucocyte function associated antigen-1, LFA-1) {35}, and GXM can induce shedding of neutrophil L-selectin {36}, a molecule that is involved in the first steps of endothelial leukocyte transmigration.
GXM is easily purified {37}, and, in its purified form, it is poorly immunogenic {38}. Moreover, patients with cryptococcal meningitis can display relatively mild symptoms for a prolonged period of time despite detectable levels of GXM (in blood and CSF) {27}, probably indicating that GXM by itself is non-toxic. Therefore, GXM is a potent therapeutic agent. Of course functional equivalents of GXM will be capable of the same interference with IL-8 or otherwise induced migration of neutrophils. Thus GXM or a functional equivalent thereof can be applied in the treatment of diseases or pathological conditions involving neutrophil migration. Typically such diseases include infections or other inflammatory diseases. GXM will be especially useful in diseases which involve IL-8 regulation. Examples thereof are brain inflammatory diseases such as meningitis, in particular bacterial meningitis, and neurotrauma or the protection of the brain against toxic medication administered systemically (eg anti-cancer drugs).

Other important applications will include the treatment of pathological conditions such as brain injury or ischemic events, as described herein. In a further embodiment the invention provides a pharmaceutical composition comprising gucuronoxylmannan or a functional equivalent thereof together with a suitable means for administration. Typically such a composition will be given systemically. The dose of GXM necessary to prevent neutrophil migration may vary, but will generally be between 5 mg/kg and 250 mg/kg, preferably between 25 mg/kg and 150 mg/kg.

The compositions may of course contain other drugs for the treatment of diseases involving neutrophil migration, such as anti-inflammatory drugs, corticosteroids or an antibiotic agent.

### The invention will be explained in more detail in the following experimental part.

### Isolation of GXM.

C. neoformans is autoclaved after growth in a chemically defined medium for 5 days. Polysaccharide is precipitated with calcium acetate and ethanol. After being dissolved in NaCl and undergoing brief ultrasonic irradiation, the polysaccharide is precipitated by differential complexation with hexadecyltrimethylammonium bromide. Purified GXM is precipitated by ethanol, dissolved, ultrasonically irradiated for 2h, centrifuged, dialyzed, and finally recovered by lyophilization. Samples are analyzed through various methods to prove that none of the purified polysaccharides contain constituents other than those known to occur in GXM (37).

### Animal toxicity.

In our experiments, we have investigated whether GXM is toxic in healthy animals.
We have demonstrated that healthy rabbits tolerated intravenous administration of GXM in doses ranging from 0.5 to 50 mg. In depth histologic examination and determination of the kinetics of GXM in these rabbits are ongoing.

### References

1 Schlech WFI, Ward JI, Band JD, et al. Bacterial meningitis in the United States, 1978 through 1981. The national bacterial meningitis surveillance study. JAMA **1985**; 253:1749-54.
2 Durand ML, Calderwood SB, Weber DJ, et al. Acute bacterial meningitis in adults: a review of 493 episodes. N Engl J Med **1993**; 328:21-8.
3 Pfister HW, Fontana A, Tauber MG, Tomasz A, Scheld WM. Mechanisms of brain injury in bacterial meningitis: workshop summary. Clin Infect Dis **1994**; 19:463-79.
4 Spellerberg B, Tuomanen EL. The pathophysiology of pneumococcal meningitis. Ann Med **1994**; 26:411-8.
5 Tuomanen EI, Saukkonen K, Sande S, Cioffe C, Wright SD. Reduction of inflammation, tissue damage, and mortality in bacterial meningitis in rabbits treated with monoclonal antibodies against adhesion-promoting receptors of leukocytes. J Exp Med **1989**; 170:959-69.
6 Saez Llorens X, Jafari HS, Severien C, et al. Enhanced attenuation of meningeal inflammation and brain edema by concomitant administration of anti-CD18 monoclonal antibodies and dexamethasone in experimental Haemophilus meningitis. J Clin Invest **1991**; 88:2003-11.
7 Cronstein BN, Kimmel SC, Levin RI, Martiniuk F, Weissmann G. A mechanism for the antiinflammatory effects of corticosteroids: the glucocorticoid receptor regulates leukocyte adhesion to endothelial cells and expression of endothelial-leukocyte adhesion molecule 1 and intercellular adhesion molecule 1. Proc Natl Acad Sci U S A **1992**; 89:9991-5.
8 Quagliarello VJ, Scheld WM. Treatment of bacterial meningitis. N Engl J Med **1997**; 336:708-16.
9 Hartl R, Medary MB, Ruge M, ArforS KE, Ghajar J. Early white blood cell dynamics after traumatic brain injury: effects on the cerebral microcirculation. J Cereb Blood Flow Metab **1997**; 17:1210-20.
10 Hallenbeck JM. Cytokines, macrophages, and leukocytes in brain ischemia. Neurology **1997**; 49:S5-9.
11 Winquist RJ, Kerr S. Cerebral ischemia-reperfusion injury and adhesion. Neurology **1997**; 49:S23-6.
12 Yanaka K, Camarata PJ, Spellman SR, McCarthy JB, Furcht LT, Low WC. Antagonism of leukocyte adherence by synthetic fibronectin peptide V in a rat model of transient focal cerebral ischemia. Neurosurgery **1997**; 40:557-63.
13 Korthuis RJ, Anderson DC, Granger DN. Role of neutrophil-endothelial cell adhesion in inflammatory disorders. J Crit Care **1994**; 9:47-71.
14 Tanaka M, Brooks SE, Richard VJ, et al. Effect of anti-CD18 antibody on myocardial neutrophil accumulation and infarct size after ischemia and reperfusion in dogs. Circulation **1993**; 87:526-35.
15 Schoenberg MH, Poch B, Younes M, et al. Involvement of neutrophils in postischemic damage to the small intestine. Gut **1991**; 32:905-12.
16 Kubes P, Hunter J, Granger DN. Ischemia/reperfusion-induced feline intestinal dysfunction: importance of granulocyte recruitment. Gastroenterology **1992**; 103:807-12.
17 Sekido N, Mukaida N, Harada A, Nakanishi I, Watanabe Y, Matsushima K. Prevention of lung reperfusion injury in rabbits by a monoclonal antibody against interleukin-8. Nature **1993**; 365:654-7.
18 Petrasek PF, Liauw S, Romaschin AD, Walker PM. Salvage of postischemic skeletal muscle by monoclonal antibody blockade of neutrophil adhesion molecule CD18. J Surg Res **1994**; 56:5-12.
19 Clark WM, Zivin JA. Antileukocyte adhesion therapy: preclinical trials and combination therapy. Neurology **1997**; 49:S32-8.
20 Tuomanen E. Adjunctive therapy of experimental meningitis: agents other than steroids. Antibiot Chemother **1992**; 45:184-91.
21 Ley K, Cerrito M, Arfors KE. Sulfated polysaccharides inhibit leukocyte rolling in rabbit mesentery venules. Am J Physiol **1991**; 260:H1667-73.
22 Ley K, Linnemann G, Meinen M, Stoolman L, Gaehtgens P. Fucoidin, but not yeast polyphosphomannan PPME, inhibits leukocyte rolling in venules of the rat mesentery. Blood **1993**; 81:177-85.
23 Adams DH, Lloyd AR. Chemokines; leukocyte recruitment and activation cytokines. Lancet **1997**; 349:490-5.
24 Taub DD. Chemokine-leukocyte interactions: the voodoo that they do so well. Cytokine Growth Factor Reviews **1996**; 7:355-76.
25 Bell MD, Taub DD, Perry VH. Overriding the brain's intrinsic resistance to leukocyte recruitment with intraparenchymal injections of recombinant chemokines. Neuroscience **1996**; 74:283-92.
26 Chaka WS, Heyderman R, Gangaidzo I, et al. Cytokine profiles in CSF of HIV-infected patients with cryptococcal meningitis: no leukocytosis despite high IL-8 levels. J Infect Dis **1997**; 176:1633-6.
27 Mitchell TG, Perfect JR. Cryptococcosis in the era of AIDS - 100 years after the discovery of *Cryptococcus neoformans*. Clinical Microbiology Reviews **1995**; 8:515-48.
28 Eng RHK, Bishburg E, Smith SM. Cryptococcal infections in patients with acquired immune deficiency syndrome. Am J Med **1986**; 81:19-23.
29 Drouhet E, Segretain G. Inhibition de la migration leucocytaire in vitro par un polyoside capsulaire de *Torulopsis (Cr**yptococcus**) neoformans*.. Ann Inst Pasteur **1951**; 81:674-6.
30 Dong ZM, Murphy JW. Effects of two varieties of *Cryptococcus neoformans* cells and culture filtrate antigens on neutrophil locomotion. Infect Immun **1995**; 63:2632-44.
31 Dong ZM, Murphy JW. Intravascular cryptococcal culture filtrate (CneF) and its major component, glucuronoxylomannan, are potent inhibitors of leukocyte accumulation. Infect Immun **1995**; 63:770-8.
32 Dong ZM, Murphy JW. Mobility of human neutrophils in response to Cryptococcus neoformans cells, culture filtrate antigen, and individual components of the antigen. Infect Immun **1993**; 61:5067-77.
33 Lipovsky MM, Gekker G, Hu S, Ehrlich LC, Hoepelman AIM, Peterson PK. Cryptococcal glucuronoxylomannan induces interleukin (IL)-8 production by human microglia but inhibits neutrophil migration towards IL-8. J Infect Dis **1998**; 177:260-3.
34 Cherniak R, Sundstrom JB. Polysaccharide antigens of the capsule of Cryptococcus neoformans. Infect Immun **1994**; 62:1507-12.
35 Dong ZM, Murphy JW. Cryptococcal polysaccharides bind to CD18 on human neutrophils. Infect Immun **1997**; 65:557-63.
36 Dong ZM, Murphy JW. Cryptococcal polysaccharides induce L-selectin shedding and tumor necrosis factor receptor loss from the surface of human neutrophils. J Clin Invest **1996**; 97:689-98.
37 Cherniak R, Morris LC, Anderson BC, Meyer SA. Facilitated isolation, purification, and analysis of glucuronoxylomannan of *Cryptococcus neoformans*.. Infect Immun **1991**; 59:59-64.
38 Eckert TF, Kozel TR. Production and characterization of monoclonal antibodies specific for *Cryptococcus neoformans* capsular polysaccharide. Infect Immun **1987**; 55:1895-9.
39 Burroughs MH, Tsenova Berkova L, Sokol K, Ossig J, Tuomanen E, Kaplan G. Effect of thalidomide on the inflammatory response in cerebrospinal fluid in experimental bacterial meningitis. Microb Pathog **1995**; 19:245-55.

## Claims

1. Glucuronoxylomannan or a functional equivalent thereof for use as a pharmaceutical.

2. Use of glucuronoxylmannan in the preparation of a medicament for the treatment of a pathological condition or a disease involving neutrofil migration.

3. Use according to claim 2 whereby the disease is an infection or an inflammatory disease.

4. Use according to claim 3, whereby the inflammatory disease involves IL-8 regulation.

5. Use according to anyone of claims 2-4, whereby the inflammatory disease is meningitis, in particular bacterial meningitis.

6. Use according to claim 2 whereby the pathological condition is serious brain injury or an ischemic event.

7. Use according to claim 6 whereby the ischemic event is a myocardial infarct, cerebrovascular ischemia, intestinal ischemia, pulmonary ischemia or skeletal muscular ischemia.

8. A pharmaceutical composition comprising gucuronoxylmannan or a functional equivalent thereof together with a suitable means for administration.

9. A pharmaceutical composition according to claim 8, further comprising a further anti-inflammatory drug.

10. A pharmaceutical composition according to claim 8 or 9, further comprising a corticosteroid.

11. A pharmaceutical composition according to any one of claims 9-10, further comprising an antibiotic agent.
